# EUROPEAN PATENT APPLICATION

(11) **EP 3 520 676 A1**
(43) Date of publication of application: **07.08.2019**
(21) Application number: 17855392.1
(22) Date of filing: 25.07.2017
(51) Int. Cl.: A61B 1/045, A61B 1/00

(54) **PROCESSOR DEVICE, ENDOSCOPE SYSTEM, AND OPERATION METHOD FOR PROCESSOR DEVICE**

(30) Priority: 30.09.2016 JP 2016192892
(71) Applicant: FUJIFILM Corporation, Tokyo 106-8620 (JP)
(72) Inventor: ENDO Maiko, Ashigarakami-gun Kanagawa 258-8538 (JP)
(74) Representative: Klunker IP Patentanwälte PartG mbB
(86) International application number: PCT/JP2017/026814
(87) International publication number: WO 2018/061434

(57) **Abstract**

Provided are a processor device, an endoscope system, and a method of operating a processor device capable of calculating only an index value capable of being calculated from an image acquired in a specific situation.

An image obtained by imaging an observation target including a structure is acquired. A clarity calculation unit (80) calculates a clarity of the structure on the basis of the image. An index value selection unit (82) selects an index value capable of being calculated on the basis of the clarity of the structure calculated by the clarity calculation unit (80) among a plurality of index values. An index value calculation unit (84) calculates the index value selected by the index value selection unit (82), on the basis of the image.

## Description

### BACKGROUND OF THE INVENTION

### 1. Field of the Invention

The present invention relates to a processor device, an endoscope system, and a method of operating a processor device, which calculate index values obtained by indexing living body features of an observation target.

### 2. Description of the Related Art

In the medical field, diagnosis using an endoscope system comprising a light source device, an endoscope, and a processor device has been performed widely. The endoscope system irradiates an observation target via the endoscope with illumination light from the light source device, and the processor device produces an image of the observation target on the basis of image signals obtained by capturing the observation target under illumination with the illumination light. By displaying the image on a monitor, a doctor can perform diagnosis while viewing this image on the monitor.

Additionally, in recent years, index values obtained by indexing living body features of an observation target, such as a blood vessel structure or a glandular structure, are also used in a case where a lesion is diagnosed. For example, in JP2016-116741A, blood vessel index values, such as blood vessel thickness, blood vessel length, and blood vessel density, are calculated as the index values, and are displayed on a monitor or the like. Additionally, in JP2016-116741A, the blood vessel index values are accurately calculated by performing correction in accordance with the observation distance with respect to the blood vessel thickness and the blood vessel length that vary depending on the observation distances even among the blood vessel index values.

### SUMMARY OF THE INVENTION

In observation using an endoscopic image, a high-quality bright image is obtained in a situation like the noise amount of the image is small or the observation distance is short. Therefore, it is possible to calculate two or more kinds of index values. In contrast, in a situation like the noise amount is large or the observation distance is long, only a low-quality dark image is obtained. Only limited index values cannot be calculated from such a low-quality image. In this regard, JP2016-116741A discloses that all the index values can be calculated from an image obtained by imaging the observation target, as a premise, and does not disclose and suggest that an index value that cannot be calculated is present depending on the quality of an image.

An object of the invention is to provide a processor device, an endoscope system, and a method of operating a processor device capable of selecting and calculating an index value capable of being calculated from an image acquired in a specific situation.

A processor device of the invention comprises an image acquisition unit that acquires an image obtained by imaging an observation target including a structure; a clarity calculation unit that calculates a clarity of the structure on the basis of the image; an index value selection unit that selects an index value capable of being calculated on the basis of the image having the clarity of the structure calculated by the clarity calculation unit among a plurality of index values; and an index value calculation unit that calculates the index value selected by the index value selection unit, on the basis of the image.

It is preferable that the clarity of the structure is expressed by a noise amount of the image, the clarity calculation unit has a noise amount calculation unit that calculates the noise amount, and the index value selection unit selects an index value capable of being calculated from the image having the noise amount among the plurality of index values. It is preferable that the processor device further comprises gain processing unit that performs a gain processing on the image, and the noise amount calculation unit calculates the noise amount from a gain amount to be used in the gain processing. It is preferable that the noise amount calculated by the noise amount calculation unit is changed in accordance with a modulated light signal to be used for control of a light source in a case where a quantity of light emission of the light source that emits the light with which the observation target is illuminated exceeds a specific quantity of light emission. It is preferable that the noise amount calculation unit calculates the noise amount from a spatial change amount of a pixel value of the image. It is preferable that the noise amount calculation unit calculates the noise amount from a temporal change amount of a pixel value of the image.

It is preferable that the index value selection unit selects an index value for a high noise including an index value relating to a low-frequency structure in a case where the noise amount exceeds the specific noise amount threshold value, and selects an index value for a low noise including an index value relating to a high-frequency structure in a case where the noise amount is equal to or less than the specific noise amount threshold value. It is preferable that the index value for a high noise includes an area of the structure or a density of the structure, and the index value for a low noise includes a thickness of the structure or a length of the structure.

It is preferable that the clarity of the structure is expressed by an observation distance indicating a distance from the observation target, the clarity calculation unit has an observation distance calculation unit that calculates the observation distance on the basis of the image, and the index value selection unit selects an index value capable of being calculated from the image obtained in the calculated observation distance among the plurality of index values. It is preferable that the index value selection unit selects an index value for a first observation distance including an index value relating to a low-frequency structure in a case where the observation distance is a first observation distance exceeding a specific observation distance threshold value, and selects an index value for a second observation distance including an index value relating to a high-frequency structure in a case where the observation distance is a second observation distance equal to or less than the specific observation distance threshold value. It is preferable that the index value for a first observation distance includes an area of the structure or a density of the structure, and the index value for a second observation distance includes a thickness of the structure or a length of the structure. It is preferable that the structure is a blood vessel structure or a glandular structure.

A processor device of the invention comprises an image acquisition unit that acquires an image obtained by imaging an observation target including a structure; a noise amount calculation unit that calculates a noise amount of the image on the basis of the image; an index value selection unit that selects an index value capable of being calculated from the image having the noise amount among a plurality of index values, the index value selection unit selecting an index value for a high noise including an index value relating to a low-frequency structure in a case where the noise amount exceeds a specific noise amount threshold value, and selecting an index value for a low noise including an index value relating to the high-frequency structure in a case where the noise amount is equal to or less than the specific noise amount threshold value; and an index value calculation unit that calculates an index value selected by the index value selection unit, on the basis of the image.

An endoscope system of the invention comprises an image acquisition unit that acquires an image obtained by imaging an observation target including a structure; a clarity calculation unit that calculates a clarity of the structure on the basis of the image; an index value selection unit that selects an index value capable of being calculated on the basis of the clarity of the structure calculated by the clarity calculation unit among a plurality of index values; an index value calculation unit that calculates the index value selected by the index value selection unit, on the basis of the image; an index value image generation unit that generates an index value image obtained by imaging the index value; and a display unit that displays the index value image.

A method of operating a processor device of the invention comprises the steps of acquiring an image obtained by imaging an observation target including a structure, using an image acquisition unit; calculating a clarity of the structure on the basis of the image, using a clarity calculation unit; selecting an index value capable of being calculated on the basis of the clarity of the structure calculated by the clarity calculation unit among a plurality of index values, using index value selection unit; and calculating the index value selected by the index value selection unit, on the basis of the image, using an index value calculation unit.

According to the invention, it is possible to select and calculate the index value capable of being calculated from the image acquired in the specific situation.

### BRIEF DESCRIPTION OF THE DRAWINGS

Fig. 1 is an external view of an endoscope system.
Fig. 2 is a block diagram illustrating the functions of the endoscope system of the first embodiment.
Fig. 3 is a graph illustrating the spectroscopic spectrum of violet light V, blue light B, blue light Bx, green light G, and red light R.
Fig. 4 is a graph illustrating the spectroscopic spectrum of normal light of the first embodiment.
Fig. 5 is a graph illustrating the spectroscopic spectrum of special light of the first embodiment.
Fig. 6 is a block diagram illustrating the functions of an index value image processing unit comprising a noise amount calculation unit.
Fig. 7 is an explanatory view illustrating a noise amount conversion table.
Fig. 8 is an image view illustrating a thin surface layer blood vessel V1 and a thick middle depth layer blood vessel V2.
Fig. 9 is an explanatory view illustrating an index value classification table.
Fig. 10 is an image view illustrating a blood vessel density image, a blood vessel area image, and a blood vessel complexity image in which numerical index values are displayed laterally.
Fig. 11 is an image view illustrating the blood vessel density image, the blood vessel area image, and the blood vessel complexity image that are displayed in parallel.
Fig. 12 is an explanatory view illustrating that three kinds of images of the blood vessel density image, the blood vessel area image, and the blood vessel complexity image are switched and displayed.
Fig. 13 is a flowchart illustrating an index value display mode.
Fig. 14 is a block diagram illustrating the functions of the index value image processing unit comprising an observation distance calculation unit.
Fig. 15 is a block diagram illustrating the functions of an endoscope system of a second embodiment.
Fig. 16 is a graph illustrating the spectroscopic spectrum of normal light of the second embodiment.
Fig. 17 is a graph illustrating the spectroscopic spectrum of special light of the second embodiment.
Fig. 18 is a block diagram illustrating the functions of an endoscope system of a third embodiment.
Fig. 19 is a plan view of a rotation filter.

### DESCRIPTION OF THE PREFERRED EMBODIMENTS

### [First Embodiment]

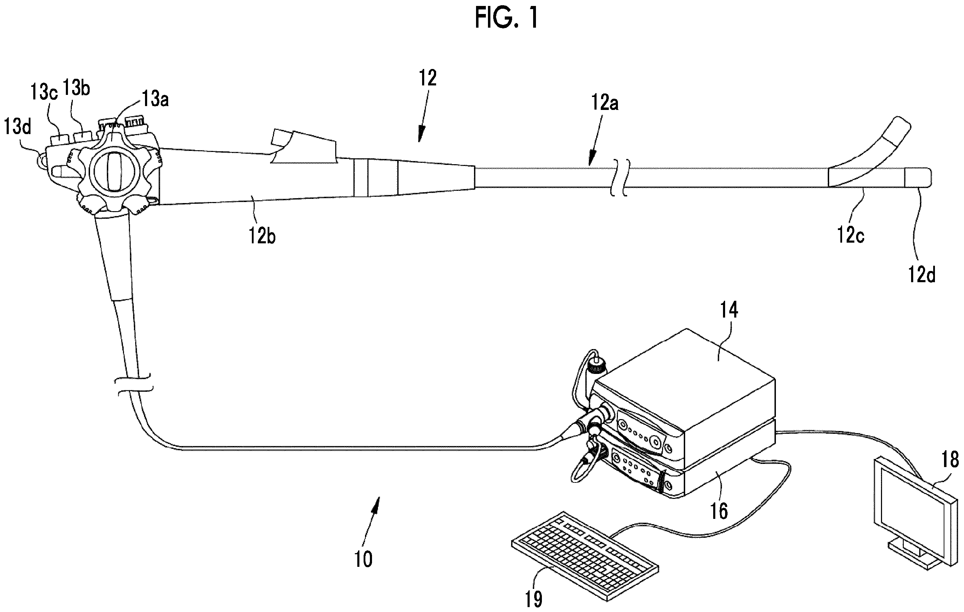

As illustrated in Fig. 1, an endoscope system 10 has an endoscope 12, a light source device 14, a processor device 16, a monitor 18 (display unit), and a console 19. The endoscope 12 is optically connected to the light source device 14, and is electrically connected to the processor device 16. The endoscope 12 has an insertion part 12a to be inserted into a subject, an operating part 12b provided at a proximal end portion of the insertion part 12a, and a bending part 12c and a distal end part 12d provided on a distal end side of the insertion part 12a. By operating an angle knob 13a of the operating part 12b, the bending part 12c makes a bending motion. The distal end part 12d is directed in a desired direction by this bending motion.

Additionally, the operating part 12b is provided with a still image acquisition unit 13b used for operating the acquisition of still images, a mode switching unit 13c used for operating the switching of observation modes, and a zooming operating unit 13d used for operating the change of a zoom magnification factor, in addition to the angle knob 13a. In the still image acquisition unit 13b, a freeze operation of displaying a still image of an observation target on the monitor 18, and a release operation of saving the still image in a storage are possible.

The endoscope system 10 has a normal mode, a special mode, and an index value display mode as the observation modes. In a case where an observation mode is the normal mode, normal light obtained by combining a plurality of colors of light components together in a quantity-of-light ratio Lc for normal mode is emitted, and a normal image is displayed on a monitor 18 on the basis of image signals obtained by imaging the observation target under illumination with normal light. Additionally, in a case where an observation mode is the special mode, special light obtained by combining a plurality of colors of light components together in a quantity-of-light ratio Ls for special mode is emitted, and a special image is displayed on the monitor 18 on the basis of image signals obtained by imaging the observation target under illumination with special light. Additionally, in a case where an observation mode is the index value display mode, the special light is emitted, and an index value image relating to the index values obtained by indexing the living body features of the observation target is displayed on the monitor 18 on the basis of image signals obtained by imaging the observation target under illumination with special light.

The processor device 16 is electrically connected to the monitor 18 and the console 19. The monitor 18 outputs and displays an image of the observation target, information accompanying the image, or the like. The console 19 functions as a user interface that receives input operations, such as designation or the like of a region of interest (ROI) or function setting.

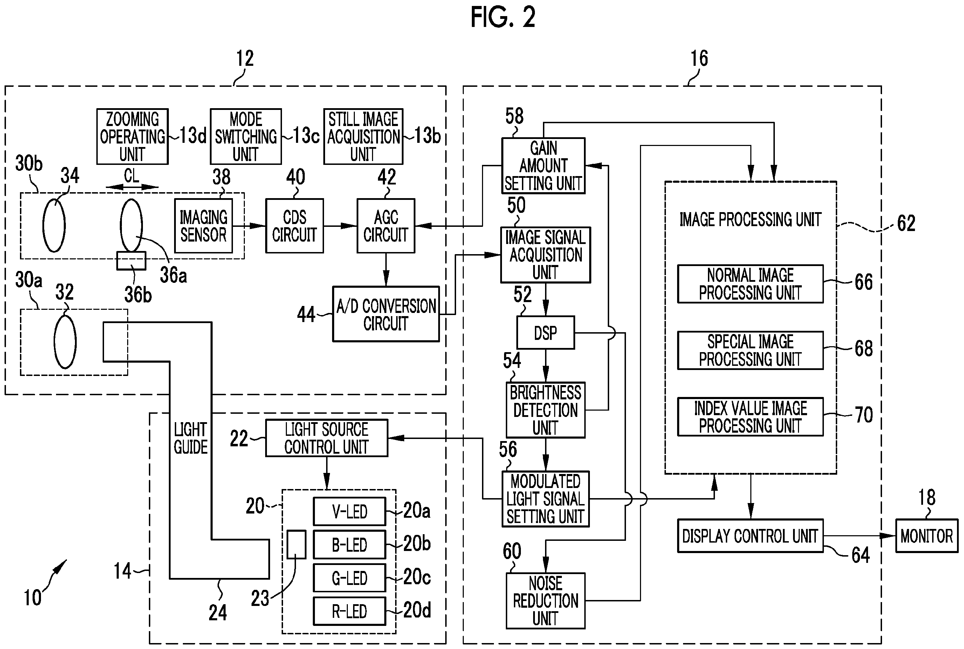

As illustrated in Fig. 2, the light source device 14 comprises a light source 20 that emits the illumination light to be used for illumination of the observation target, and a light source control unit 22 that controls the light source 20. The light source 20 is semiconductor light sources, such as a plurality of colors of light-emitting diodes (LEDs). The light source control unit 22 controls the quantity of light emission of the illumination light by ON/OFF of the LEDs and the adjustment of the driving currents or driving voltages of the LEDs. Additionally, the light source control unit 22 controls the wavelength range of the illumination light, for example, by changing the optical filters.

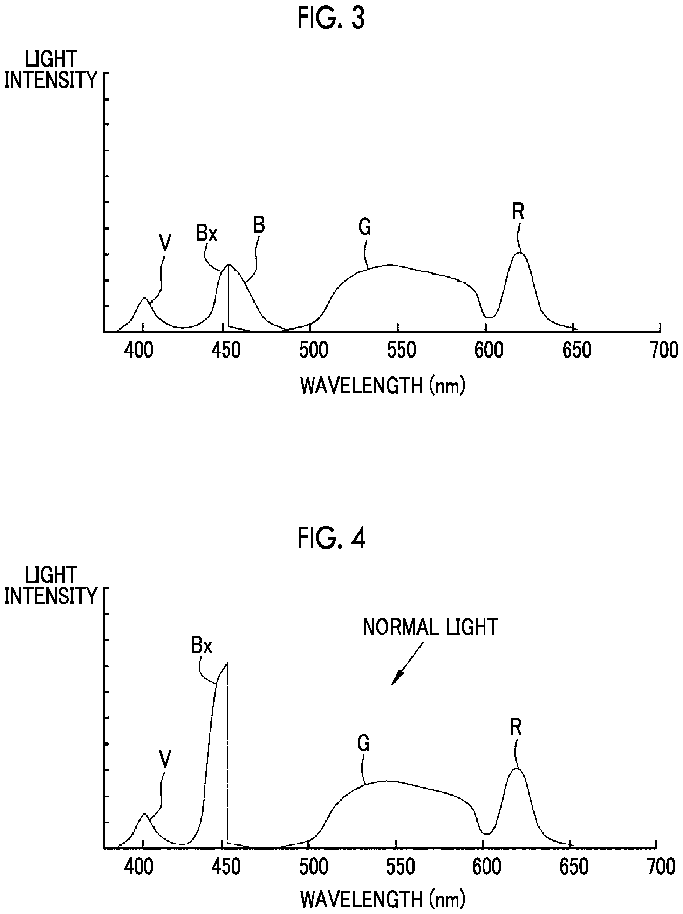

In the first embodiment, the light source 20 has four color LEDs of a violet light-emitting diode (V-LED) 20a, a blue light-emitting diode (B-LED) 20b, a green light-emitting diode (G-LED) 20c, a red light-emitting diode (R-LED) 20d, and a wavelength cutoff filter 23. As illustrated in Fig. 3, the V-LED 20a emits violet light V having a wavelength range of 380 nm to 420 nm.

The B-LED 20b emits blue light B having a wavelength range of 420 nm to 500 nm. The blue light B emitted from the B-LED 20b is cut by the wavelength cutoff filter 23 on at least a longer wavelength side than the peak wavelength 460 nm. Accordingly, the blue light Bx after being transmitted through the wavelength cutoff filter 23 has a wavelength range of 420 to 460 nm. In this way, the reason why light in a wavelength range on the longer wavelength side than 460 nm is cut is that the light in the wavelength range on the longer wavelength side than 460 nm is a factor in which the blood vessel contrast of blood vessels that is the observation target is lowered. In addition, the wavelength cutoff filter 23 may reduce the light in the wavelength range on the longer wavelength side than 460 nm instead of cutting the light in the wavelength range on the longer wavelength side than 460 nm.

The G-LED 20c emits green light G having a wavelength range of 480 nm to 600 nm. The R-LED 20d emits red light R having a wavelength range of 600 nm to 650 nm. In addition, center wavelengths and peak wavelengths of the respective light of colors emitted from the LEDs 20a to 20d may be the same as each other or may be different from each other.

The light source control unit 22 independently controls ON/OFF of the respective LEDs 20a to 20d, the quantity of light emission at the time of ON, and the like, thereby adjusting the light emission timing of illumination light, a light emission period, light intensity, and a spectroscopic spectrum. In a case where the quantity of light emission of the illumination light is controlled, the light source control unit 22 controls the LEDs 20a to 20d such that the observation target has a reference brightness, on the basis of a modulated light signal set in a modulated light signal setting unit 56 of the processor device 16. The control of ON and OFF by the light source control unit 22 varies in the respective observation modes. In addition, the reference brightness is capable of being set by a brightness setting unit (not illustrated), the console 19, or the like of the light source device 14.

In the case of the normal mode, the light source control unit 22 turns on the V-LED 20a, the B-LED 20b, the G-LED 20c, and the R-LED 20d altogether. In that case, as illustrated in Fig. 4, the quantity-of-light ratio Lc between the violet light V, the blue light Bx, the green light G, and the red light R is set such that the light intensity of the blue light Bx becomes larger than the light intensity of any of the violet light V, the green light G, and the red light R. Accordingly, in the normal mode, multicolor light for normal mode including the violet light V, the blue light Bx, the green light G, and the red light R is emitted as the normal light from the light source device 14. The normal light is substantially white because respective light intensities of the violet light V, the blue light Bx, the green light G, and the red light R are equal to or more than a given value.

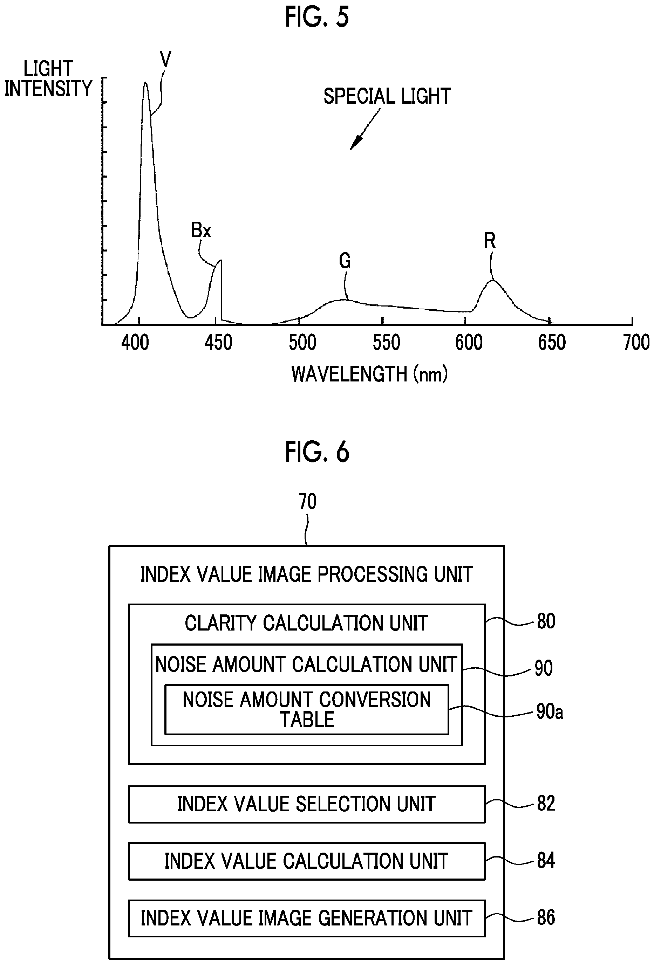

Even in the case of the special mode or the index value display mode, the light source control unit 22 turns on the V-LED 20a, the B-LED 20b, the G-LED 20c, and the R-LED 20d altogether. In that case, as illustrated in Fig. 5, the quantity-of-light ratio Ls between the violet light V, the blue light Bx, the green light G, and the red light R is set such that the light intensity of the violet light V becomes larger than the light intensity of any of the blue light Bx, the green light G, and the red light R and such that the green light G and the red light R become smaller than the violet light V and the blue light Bx. Accordingly, in the special mode or the index value display mode, multicolor light for special mode or index value display mode including the violet light V, the blue light Bx, the green light G, and the red light R is emitted as the special light from the light source device 14. Since the light intensity of the violet light V is large, the special light is bluish light.

As illustrated in Fig. 2, the illumination light emitted from the light source 20 enters a light guide 24 inserted into the insertion part 12a via a light path coupling part (not illustrated) formed with a mirror, a lens, or the like. The light guide 24 is built in the endoscope 12 and a universal cord, and propagates the illumination light up to the distal end part 12d of the endoscope 12. The universal cord is a cord that connects the endoscope 12, and the light source device 14 and the processor device 16 together. In addition, multimode fiber can be used as the light guide 24. As an example, a fine-diameter fiber cable of which the core diameter is 105 µm, the clad diameter is 125 µm, and a diameter including a protective layer used as an outer cover is φ0.3 mm to 0.5 mm can be used for the light guide 24.

The distal end part 12d of the endoscope 12 is provided with an illumination optical system 30a and an imaging optical system 30b. The illumination optical system 30a has an illumination lens 32. The observation target is illuminated with the illumination light propagated through the light guide 24 via the illumination lens 32. The imaging optical system 30b has an objective lens 34, a magnifying optical system 36, and an imaging sensor 38. Various kinds of light, such as reflected light from the observation target, scattered light, and fluorescence, enters the imaging sensor 38 via the objective lens 34 and the magnifying optical system 36. Accordingly, the image of the observation target is formed on the imaging sensor 38.

The magnifying optical system 36 comprises a zoom lens 36a that magnifies the observation target, and a lens drive unit 36b that moves the zoom lens 36a in an optical axis direction CL. The zoom lens 36a magnifies or reduces the observation target of which the image is formed on the imaging sensor 38 by freely moving between a telephoto end and a wide end in accordance with a zoom control performed by the lens drive unit 36b.

The imaging sensor 38 is a color imaging sensor that images the observation target irradiated with the illumination light. Each pixel of the imaging sensor 38 is provided with any one of a red (R) color filter, a green (G) color filter, or a blue (B) color filter. The imaging sensor 38 receives light of which the wavelength range ranges from violet to blue with the B pixel provided with the B color filter, receives green light with a G pixel provided with the G color filter, and receives red light with an R pixel provided with the R color filter. Image signals of respective RGB colors are output from the respective color pixels. The imaging sensor 38 transmits the output image signals to a CDS circuit 40.

In the normal mode, the imaging sensor 38 images the observation target illuminated with the normal light, thereby outputting a Bc image signal from the B pixel, outputting a Gc image signal from the G pixel, and outputting an Rc image signal from the R pixel. Additionally, in the special mode or the index value display mode, the imaging sensor 38 images the observation target illuminated with the special light, thereby outputting a Bs image signal from the B pixel, outputting a Gs image signal from the G pixel, and outputting an Rs image signal from the R pixel.

As the imaging sensor 38, a charge coupled device (CCD) imaging sensor, a complementary metal-oxide semiconductor (CMOS) imaging sensor, or the like is available. Additionally, instead of the imaging sensor 38 provided with the color filters in the primary colors of RGB, a complementary color imaging sensor comprising complementary color filters in C (cyan), M (magenta), Y (yellow), and G (green) may be used. In a case where the complementary color imaging sensor is used, image signals of four colors of CMYG are output. For this reason, the same respective RGB image signals as those in the imaging sensor 38 can be obtained by converting the image signals of four colors of CMYG into image signals of three colors of RGB through color conversion between complementary colors and the primary colors. Additionally, instead of the imaging sensor 38, a monochrome sensor that is not provided with the color filters may be used.

The CDS circuit 40 performs correlated double sampling (CDS) on analog image signals received from the imaging sensor 38. The image signals that have passed through the CDS circuit 40 are input to the AGC circuit 42. The AGC circuit 42 performs an automatic gain control (AGC) on the input image signals. In the AGC circuit 42 (corresponding to a "gain processing unit" of the invention), in a case where the automatic gain control is performed, gain processing is performed on the image signals on the basis of the gain amount set by a gain amount setting unit 58 within the processor device 16. An analog-to-digital (A/D) conversion circuit 44 converts the analog image signals, which have passed through the AGC circuit 42, into digital image signals. The A/D conversion circuit 44 inputs the digital image signals after the A/D conversion to the processor device 16.

The processor device 16 comprises an image signal acquisition unit 50, a digital signal processor (DSP) 52, a brightness detection unit 54, the modulated light signal setting unit 56, the gain amount setting unit 58, a noise reduction unit 60, an image processing unit 62, and a display control unit 64.

The image signal acquisition unit 50 (equivalent to the "image acquisition unit" of the invention) acquires an image obtained by imaging the observation target including structure. The image signal acquisition unit 50 acquires the digital image signals corresponding to an observation mode from the endoscope 12. In the case of the normal mode, the Bc image signal, the Gc image signal, and the Rc image signal are acquired. In the case of the special mode or the index value display mode, the Bs image signal, the Gs image signal, and the Rs image signal are acquired.

The DSP 52 performs various kinds of signal processing, such as defect correction processing, offset processing, DSP gain correction processing, linear matrix processing, gamma conversion processing, demosaicing processing, and the like, on the image signals acquired by the image signal acquisition unit 50. In the defect correction processing, a signal of a defective pixel of the imaging sensor 38 is corrected. In the offset processing, a dark current component is removed from the image signals subjected to the defect correction processing, and an accurate zero level is set. In the DSP gain correction processing, a signal level is adjusted by multiplying the image signals subjected to the offset processing by a specific DSP gain.

The linear matrix processing enhances color reproducibility on the image signals subjected to the DSP gain correction processing. In the gamma conversion processing, brightness and saturation of image signals subjected the linear matrix processing are adjusted. By performing the demosaicing processing (also referred to as equalization processing or synchronization processing) on the image signals subjected to the gamma conversion processing, a signal of a color that runs short in each pixel is generated by interpolation. By means of this demosaicing processing, all pixels have signals of respective RGB colors.

The brightness detection unit 54 detects the brightness of the observation target on the basis of the image signals output from the DSP 52. For example, the brightness detection unit 54 detects the brightness of the observation target by calculating an average value of signal values of the image signals. The modulated light signal setting unit 56 sets the modulated light signal used for the control of each of light source that constitute the LEDs 20a to 20d. The modulated light signal setting unit 56 sets the modulated light signal on the basis of the brightness of the observation target detected by the brightness detection unit 54 and the reference brightness. The modulated light signal is set on the basis of a difference between the brightness of the observation target and the reference brightness, and increases as the modulated light signal is away the reference brightness. The set modulated light signal is input to the light source control unit 22. The light source control unit 22 controls the quantity of light emission of each of the LEDs 20a to 20d such that the reference brightness is obtained.

The gain amount setting unit 58 sets the amount of gain on the basis of the brightness of the observation target detected by the brightness detection unit 54 and the reference brightness. The gain amount is set on the basis of the difference between the brightness of the observation target and the reference brightness, and increases as the gain amount is away the reference brightness. The set gain amount is input to the AGC circuit 42. By performing the gain processing based on the gain amount on the image signals, the AGC circuit 42 adjusts the signal values of the image signals so as to have signal values with the reference brightness. The noise reduction unit 60 performs noise reducing processing using, for example, a moving average method, a median filter method, or the like on the image signals subjected to the demosaicing processing or the like by the DSP 52, and reduces noise. The image signals after the noise reduction are input to the image processing unit 62.

The image processing unit 62 comprises a normal image processing unit 66, a special image processing unit 68, and an index value image processing unit 70. The normal image processing unit 66 operates in a case where the normal mode is set, and performs color conversion processing, color enhancement processing, and structure enhancement processing on the received Bc image signal, Gc image signal, and Rc image signal. In the color conversion processing, color conversion processing is performed on the RGB image signals by 3x3 matrix processing, gradation transformation processing, three-dimensional look-up table (LUT) processing, and the like.

The color enhancement processing is performed on the RGB image signals subjected to the color conversion processing. The structure enhancement processing is the processing of enhancing the structure of the observation target, and is performed on the RGB image signals after the color enhancement processing. The normal image is obtained by performing the various kinds of image processing as described above. Since the normal image is an image obtained on the basis of the normal light in which the light intensity of each of the violet light V, the blue light Bx, the green light G, and the red light R is equal to or more than a given value, the normal image is a natural-tone image. The normal image is input to the display control unit 64.

The special image processing unit 68 operates in a case where the special mode is set. The special image processing unit 68 performs the color conversion processing, the color enhancement processing, and the structure enhancement processing on the received Bs image signal, Gs image signal, and Rs image signal. The processing contents of the color conversion processing, the color enhancement processing, and the structure enhancement processing are the same as the processing contents in the normal image processing unit 66. The special image is obtained by performing the various kinds of image processing as described above. Since the special image is an image obtained on the basis of special light in which the violet light V with a high absorption coefficient of hemoglobin of blood vessels has a larger light intensity than the blue light Bx, the green light G, and the red light R in the other colors, the resolution of a blood vessel structure or a glandular structure is higher than that of the other structures. The special image is input to the display control unit 64.

The index value image processing unit 70 operates in a case where the index value display mode is set. The index value image processing unit 70 calculates index values of various structures of the observation target including the blood vessel structure or the glandular structure on the basis of the received Bs image signal, Gs image signal, and Rs image signal. The index value image processing unit 70 is capable of circulating a plurality of index values. Additionally, the index value image processing unit 70 selects an index value capable of being calculated depending on the clarity of the various structures among the plurality of index values. Additionally, the index value image processing unit 70 generates the index value image on the basis of the calculated index values. The details of the index value image processing unit 70 will be described below. The index value image is input to the display control unit 64.

The display control unit 64 performs a display control for displaying an image input from the image processing unit 62 on the monitor 18. In a case where the normal mode, the display control unit 64 performs the control of displaying the normal image on the monitor 18. In a case where the special mode is set, the display control unit 64 performs the control of displaying the special image on the monitor 18. In a case where the index value display mode is set, the display control unit 64 performs the control of displaying the index value image on the monitor 18.

As illustrated in Fig. 6, the index value image processing unit 70 comprises a clarity calculation unit 80, an index value selection unit 82, an index value calculation unit 84, and an index value image generation unit 86. The clarity calculation unit 80 calculates the clarity of a structure on the basis of an image acquired by the image signal acquisition unit 50. The clarity calculation unit 80 calculates the clarity of a structure, such as the blood vessel structure or the glandular structure. The clarity of a structure means the clarity of a structure displayed as an image on the monitor 18. Since the clarity of a structure varies with the noise amount of an image, the clarity of the structure is expressed by the noise amount of the image. For this reason, the clarity calculation unit 80 comprises a noise amount calculation unit 90 that calculates the noise amount of an image.

The noise amount calculation unit 90 is capable of performing three kinds of first to third noise calculation processings, and calculates the noise amount by any one processing of the first to third noise calculation processings. In addition, a plurality of noise amounts may be calculated by performing all or some of the first to third noise calculation processings, and the plurality of noise amounts may be weighted and added.

In the first noise calculation processing, the noise amount calculation unit 90 calculates the noise amount on the basis of the gain amount set in the gain amount setting unit 58. In a case where the first noise amount calculation processing is performed, the noise amount calculation unit 90 acquires the gain amount with reference to the gain amount setting unit 58. A noise amount conversion table 90a in which the gain amount and the noise amount are stored in association with each other is used for the calculation of the noise amount based on the gain amount.

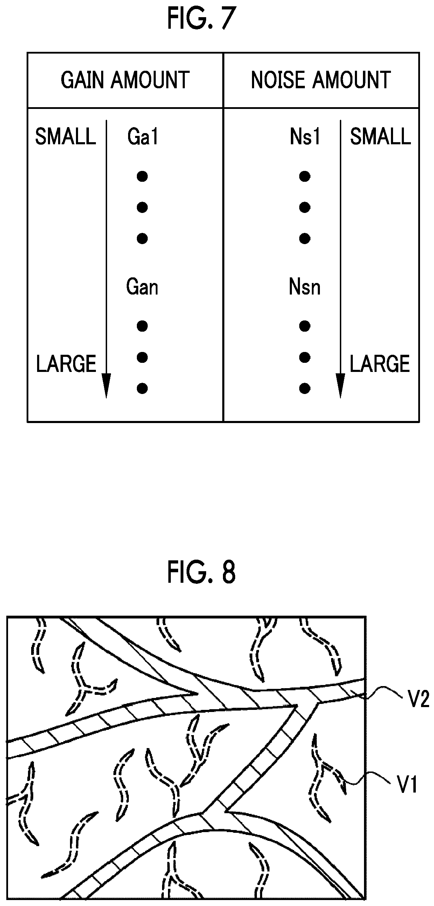

In the noise amount conversion table 90a, as illustrated in Fig. 7, a noise amount Nsl is associated with a gain amount Ga1, and a noise amount Nsn larger than the noise amount Ns1 is associated with a gain amount Gan larger than the gain amount Ga1. Hence, in the noise amount conversion table 90a, the association between the gain amount and the noise amount is performed such that, as the gain amount increases, the noise amount also increases. This is because, in a case where the gain amount is large, the brightness of a portion of the observation target contributing to diagnosis can be made bright, while the brightness of a noise component unnecessary for the diagnosis also increases.

In addition, in the first noise calculation processing, in a case where the quantity of light emission of a light source exceeds a specific quantity of light emission, the calculated noise amount may be changed in accordance with the modulated light signal set in the modulated light signal setting unit 56. For example, in a case where the quantity of light emission of one of at least any one LED among the LEDs 20a to 20d that constitute the light source 20 is the maximum quantity of light emission exceeding the specific quantity of light emission, the noise amount calculation unit 90 may change the calculated noise amount with reference to the modulated light signal. It is preferable that the noise amount calculation unit 90 multiplies the noise amount acquired with reference to the noise amount conversion table 90a by a specific correction coefficient. In addition, the noise amount calculation unit 90 calculates the noise amount from the modulated light signal after a relationship between the modulated light signal and the noise amount are determined in advance.

In the second noise amount calculation processing, the noise amount calculation unit 90 calculates the noise amount from the spatial change amount of the pixel value of any one image signal among the Bs image signal, the Gs image signal, or the Rs image signal. That is, the noise amount calculation unit 90 calculates the noise amount from the spatial change amount of the pixel value of the image acquired by the image signal acquisition unit 50. In a case where the noise amount is calculated by the second noise amount calculation processing, it is preferable to use image signals obtained from light with high reflectivity in the observation target. This is because the pixel values of such image signals (hereinafter referred to as "image signals of specific brightness") vary in an interlocking manner with the brightness of the observation target.

In the second noise amount calculation processing, the noise amount calculation unit 90 specifies a high-pixel-value region where pixel values are equal to or more than a given value, among the image signals of specific brightness, and measures the dispersion of the high-pixel-value region. Then, the noise amount calculation unit 90 calculates the noise amount from the measured dispersion. The noise amount calculated is larger as the dispersion of the high-pixel-value region is larger. It is preferable that a conversion table in which the dispersion of the high-pixel-value region and the noise amount are associated with each other is used for the calculation of the noise amount. In addition, in a case where the dispersion of the high-pixel-value region is measured, the dispersion of a specific region (for example, a region where pixel values are included in a low-pixel-value range) where a pixel value is a specific range may be measured in addition to all the image signals of specific brightness being measured. In addition, the dispersion of a region that a user sets with the console 19 or the like may be measured.

In the third noise amount calculation processing, the noise amount is calculated from the temporal change amount of the pixel value of any one image signal among the Bs image signal, the Gs image signal, or the Rs image signal. That is, the noise amount calculation unit 90 calculates the noise amount from the temporal change amount of the pixel value of the image acquired by the image signal acquisition unit 50. Even in a case where the noise amount is calculated by the third noise amount calculation processing, it is preferable that the image signals of specific brightness are used for the same reason as the second noise amount calculation processing. In the third noise amount calculation processing, the noise amount calculation unit 90 stores image signals of specific brightness equivalent to a plurality of frames, which are acquired between several frames before and one frame before, that is, acquired in the past, in addition to image signals of specific brightness acquired in real time, in order to calculate the temporal change amount of the pixel value.

In the third noise amount calculation processing, the noise amount calculation unit 90 calculates the temporal change amount of the pixel value from the image signals of specific brightness equivalent of several frames, which are acquired in real time and the past. The noise amount calculation unit 90 calculates the noise amount from the temporal change amount of the pixel value. The noise amount calculated is larger as the temporal change amount of the pixel value is larger. In addition, it is preferable that a conversion table in which the temporal change amount of the pixel value and the noise amount are associated with each other is used for the calculation of the noise amount. Additionally, in a case where the temporal change amount is calculated, the calculation may be performed within a set region similarly to the second noise amount calculation processing.

The index value selection unit 82 selects an index value capable of being calculated on the basis of the clarity of a structure calculated by the clarity calculation unit 80 among the plurality of index values. In a case where the clarity of a structure is expressed by the noise amount, the index value selection unit 82 selects an index value capable of being calculated on the basis of the noise amount calculated by the noise amount calculation unit 90 among the plurality of index values. The index value selection unit 82 selects an index value for a high noise capable of being calculated from the Bs image signal, the Gs image signal, and the Rs image signal having a high noise amount in a case where the noise amount is a high noise amount exceeding a specific noise amount threshold value.

It is preferable that the index value for a high noise is an index value that is not influenced by the amount of noise. The index value for a high noise includes, for example, the area of a structure or the density of a structure, such as the blood vessel area or the blood vessel density. Additionally, In a case where there is a lot of noise due to shortage or the like of the brightness of the observation target, as illustrated in the image of Fig. 8, a high-frequency structure, such as a thin surface layer blood vessel VI, among structures, may be obscured by being buried in noise. As a structure that is observable even under a situation where the brightness of the observation target is insufficient in this way, there is a low-frequency structure, such as a thick middle depth layer blood vessel V2. Additionally, the low-frequency structure includes the glandular structure or the like. An index value (for example, an index value of the middle depth layer blood vessel or the glandular structure) relating to such a low-frequency structure is also included in the index value for a high noise. The index value selection unit 82 selects the index value for a high noise including the index value relating to the low-frequency structure in a case where the noise amount exceeds the specific noise amount threshold value.

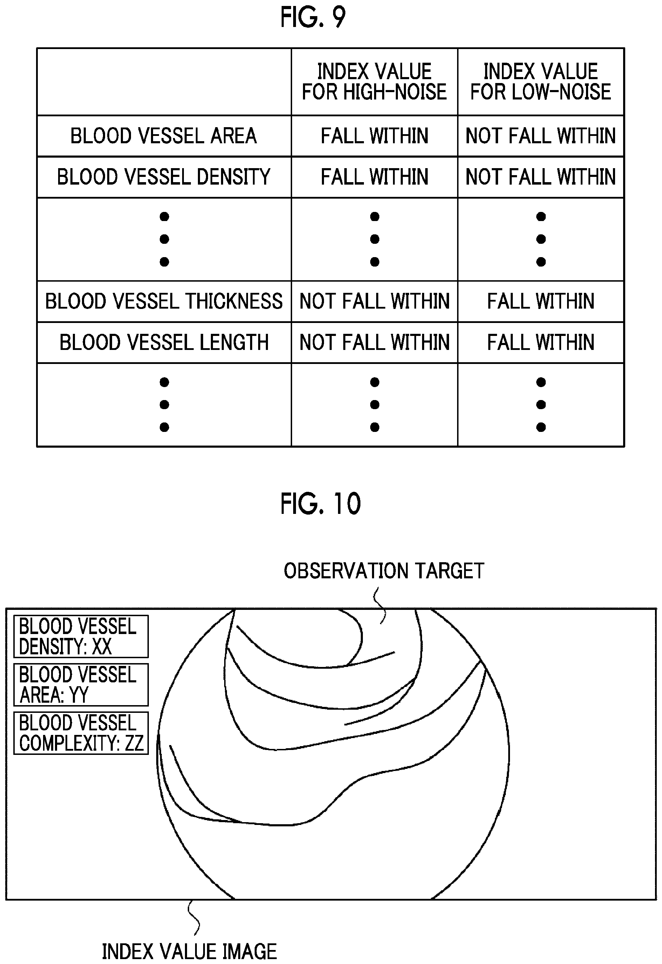

In contrast, in a case where the noise amount is a low noise amount that is equal to or less than the specific noise amount threshold value, the index value selection unit 82 selects an index value for a low noise capable of being calculated from the Bs image signal, the Gs image signal, and the Rs image signal having that has the low noise amount. It is preferable that the index value for a low noise is an index value, which is not included in the index value for a high noise, for example, an index value that is influenced in a case where there is a lot of noise. The index value for a low noise includes an index value of the high-frequency structure, such as the thin surface layer blood vessel VI, in addition to the thickness of a structure or the length of a structure. The index value selection unit 82 selects the index value for a low noise including the index value relating to the high-frequency structure in a case where the noise amount is equal to or less than the specific noise amount threshold value. In addition, in the index value selection unit 82, as illustrated in Fig. 9, it is preferable to select an index value with reference to the index value classification table to which of the index value for a high noise or the index value for a low noise the plurality of index values correspond is recorded.

The index value calculation unit 84 calculates the index value selected by the index value selection unit 82, on the basis of the image acquired by the image signal acquisition unit 50. The index value calculation unit 84 calculates the index value selected by the index value selection unit 82, on the basis of on the basis of the Bs image signal, the Gs image signal, and the Rs image signal. For example, in a case where the blood vessel density, the blood vessel area, and the blood vessel complexity (the complexity of a change in the blood vessel thickness) are selected by the index value selection unit 82, the index value calculation unit 84 extracts an image of a blood vessel on the basis of the Bs image signal, the Gs image signal, and the Rs image signal. As a method of extracting an image of a blood vessel, there is a method extracting an image of a plurality of blood vessels at different depths due to a difference between the Bs image signal and the Gs image signal. After the image of the blood vessels is extracted, the proportion of the blood vessels in unit area is calculated as the blood vessel density. Additionally, the blood vessel area is calculated by counting the pixels extracted as the blood vessels. Additionally, the blood vessel complexity is calculated by calculating the change rate of the diameter of the extracted image of the blood vessels.

In addition, in the index value calculation unit 84, the calculation may be calculated on all the index values selected by the index value selection unit 82. However, calculation may be performed only on an index value narrowed down for a diagnostic purpose set in advance by the console 19 or the like among the selected index values. For example, in a case where a diagnostic purpose is observation of a blood vessel, only an index value of the blood vessel among the selected index values is calculated such that calculation of index values other than the blood vessel, such as the glandular structure is not performed.

Additionally, in the index value calculation unit 84, a plurality of index values selected by the index value selection unit 82 may be weighted and calculated as one blood vessel parameter. It is preferable that the weighting coefficient is determined depending on the diagnostic purpose. For example, in a case where the diagnostic purpose is diagnosis of the Barrett's esophagus and a blood vessel density and the blood vessel complexity are selected, it is preferable that the blood vessel density and the blood vessel complexity are weighted and added at 1:1 and a blood vessel parameter for Barrett's esophagus is calculated.

The index value image generation unit 86 generates the index value image obtained by imaging the index values on the basis of the index values calculated by the index value calculation unit 84. As methods of generating the index value image, various image generation methods are considered. However, for example, there is generation of the index value image in which the index values are displayed as numerical values as they are. In this case, for example, in a case where the blood vessel density, the blood vessel area, and the blood vessel complexity are calculated by the index value calculation unit 84, as illustrated in Fig. 10, the index value image on which the blood vessel density, the blood vessel area, and the blood vessel complexity are displayed laterally of the observation target is generated.

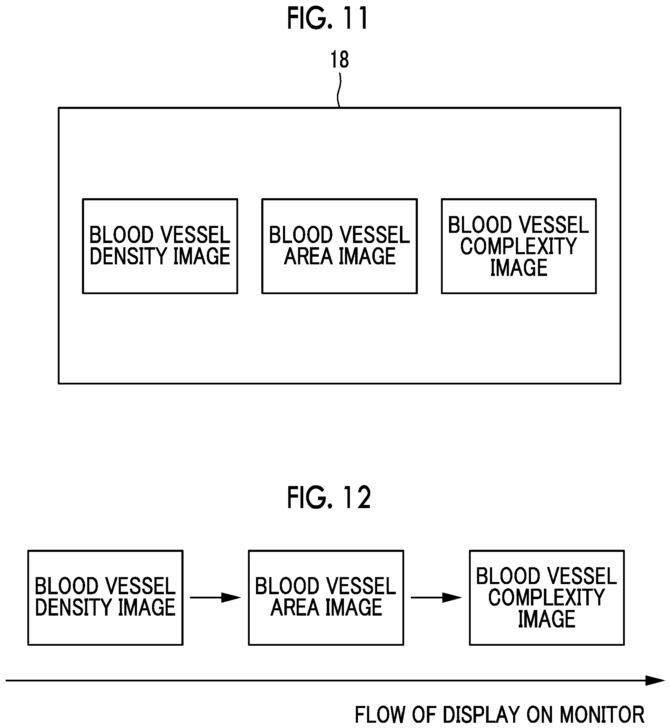

Additionally, as a method of generating the index value image, there is a method of generating the index value image displayed in different colors in accordance with the index values. In this case, for example, in a case where the blood vessel density, the blood vessel area, and the blood vessel complexity are calculated by the index value calculation unit 84, the index value image generation unit 86 generates a blood vessel density image colored in accordance with to the blood vessel density, a blood vessel area image colored in accordance with the blood vessel area, and a blood vessel complexity image colored in accordance with the blood vessel complexity. As illustrated in Fig. 11, these three blood vessel density image, blood vessel area image, and blood vessel complexity image may be displayed at one time on the monitor 18. Additionally, as illustrated in Fig. 12, the blood vessel density image, the blood vessel area image, and the blood vessel complexity image may be alternately displayed at given time intervals.

Additionally, in a case where there is a difference in accuracy depending on the clarity of a structure among the index values calculated by the index value calculation unit 84, a display pattern of the generated index value image may be changed in accordance with the clarity of the structure. For example, in a case where a plurality of index value images are switched and displayed on the monitor 18, it is preferable that the display time of an image, which is least affected by the clarity of a structure among the plurality of index value images, is made the longest, while the display time of the other images is shortened.

Additionally, as a method of generating the index value image, there is a method of generating an index value image in which the color tone of a broadband image is changed in accordance with the index values. For example, in a case where the normal image is used as the broadband image, in the index value display mode, the normal light is emitted in frames other than a frame in which the special light is emitted, and the Bc image signal, the Gc image signal, and the Rc image signal are acquired. Then, the normal image is generated on the basis of the Bc image signal, the Gc image signal, and the Rc image signal, and the index value image in which the color tone of the normal image is changed in accordance with the index values is generated by the index value image generation unit 86.

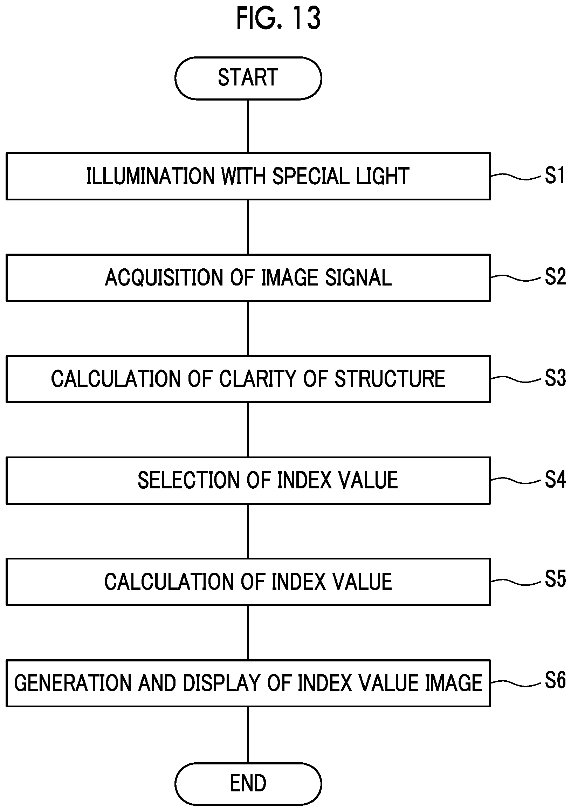

Next, the index value display mode will be described along a flowchart illustrated in Fig. 13. First, in a case where the index value display mode is set by the mode switching unit 13c, in Step S1, the observation target is irradiated with the special light. The imaging sensor 38 outputs the Bs image signal, the Gs image signal, and the Rs image signal by imaging the observation target illuminated with the special light. In Step S2, the image signal acquisition unit 50 acquires the Bs image signal, the Gs image signal, and the Rs image signal. Additionally, the brightness detection unit 54 detects the brightness of the observation target on the basis of the Bs image signal, the Gs image signal, and the Rs image signal. Additionally, the modulated light signal setting unit 56 sets the modulated light signal on the basis of the brightness of the observation target detected by the brightness detection unit 54. Additionally, the gain amount setting unit 58 sets the gain amount on the basis of the brightness of the observation target detected by the brightness detection unit 54.

Next, in Step S3, the clarity calculation unit 80 calculates the clarity of a structure. As the clarity of the structure, the noise amount calculation unit 90 calculates the noise amount. The calculation of the noise amount may be performed by any of the first to third noise amount calculation processings, using the Bs image signal, the modulated light signal, or the gain amount. After the noise amount is calculated, in Step S4, the index value selection unit 82 selects an index value capable of being calculated on the basis of the clarity of the structure calculated by the clarity calculation unit 80 among the plurality of index values. In a case where the noise amount is used as the clarity of the structure and the noise amount is the high noise amount, the index value selection unit 82 selects the index value for a high noise. On the other hand, in a case where the noise amount is the low noise amount, the index value selection unit 82 selects the index value for a low noise.

Next, in Step S5, the index value calculation unit 84 calculates the index value selected by the index value selection unit 82, on the basis of the Bs image signal, the Gs image signal, and the Rs image signal. In Step S6, the index value image generation unit 86 generates the index value image obtained by imaging the index values on the basis of the index values calculated by the index value calculation unit 84. The generated index value image is displayed on the monitor 18.

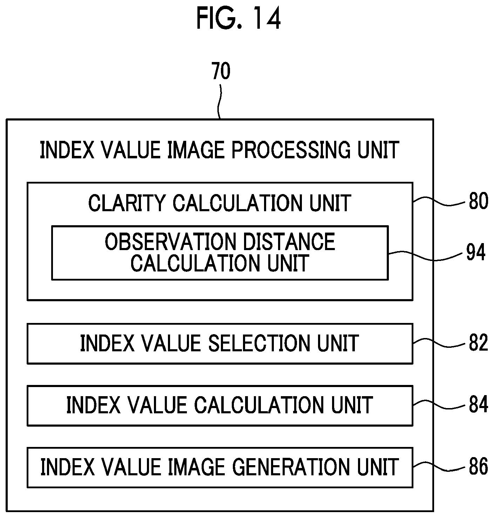

In addition, in the above embodiment, the noise amount of an image is used as the clarity of a structure. However, other variables as the clarity of the structure may be used. For example, an observation distance indicating a distance between the distal end part 12d of the endoscope and the observation target greatly influences the clarity of a structure. In a case where the observation distance is long, light does not sufficiently reach the observation target and a dark image is obtained. Therefore, the clarity of the structure becomes low. On the other hand, in a case where the observation distance is short, an obtained image is also bright and clear. Therefore, the clarity of the structure becomes high. In this way, since the clarity of the structure varies depending on the observation distance, the clarity of the structure is expressed by the observation distance indicating the distance from the observation target. In this case, as illustrated in Fig. 14, the clarity calculation unit 80 comprises the observation distance calculation unit 94 that calculates the observation distance on the basis of the image acquired by the image signal acquisition unit 50.

The observation distance calculation unit 94 calculates the observation distance on the basis of the brightness of the observation target detected by the brightness detection unit 54. The observation distance to be calculated is substantially inversely proportional to the brightness of the observation target, and the observation distance becomes smaller as the brightness of the observation target becomes larger. Additionally, the index value selection unit 82 selects an index value capable of being calculated from the Bs image signal, the Gs image signal, and the Rs image signal obtained in the calculated observation distance among the plurality of index values. That is, the index value selection unit 82 selects an index value capable of being calculated from an image obtained in the calculated observation distance among the plurality of index values.

The index value selection unit 82 selects an index value for a first observation distance capable of being calculated from the Bs image signal, the Gs image signal, and the Rs image signal obtained in a first observation distance in a case where the observation distance is the first observation distance exceeding a specific observation distance threshold value. The index value for a first observation distance includes the area of a structure or the density of the structure, which is an index value that is not easily influenced by the brightness of an image. For example, the blood vessel density or the blood vessel area is included. The index value selection unit 82 selects the index value for a first observation distance including the index value relating the low-frequency structure in a case where the observation distance is the first observation distance exceeding the specific observation distance threshold value. On the other hand, the index value selection unit 82 selects an index value for a second observation distance capable of being calculated from the Bs image signal, the Gs image signal, and the Rs image signal obtained in a second observation distance in a case where the observation distance is the second observation distance equal to or less than the specific observation distance threshold value. The index value for a second observation distance includes the thickness of a structure or the length of the structure, which is an index value that is not included in the index value for a first observation distance. For example, it is preferable that the index value for a second observation distance is an index value (the blood vessel thickness, length, or the like) that is easily influenced by the magnitude of the observation distance. The index value selection unit 82 selects the index value for a second observation distance including the index value relating the high-frequency structure in a case where the observation distance is the second observation distance equal to or less than the specific observation distance threshold value. In addition, since the method of calculating the index value and the method of generating the index value image to be performed after the index value selection are the same as those of the above embodiment using the noise amount, the description thereof will be omitted.

In addition, in the above embodiment, in a case where the observation distance becomes short, it is preferable to be able to accurately calculate the index values even in a situation where halation in which pixel values become extremely high occurs in a portion of an image region. In this case, a halation detection unit (not illustrated) is provided within the processor device 16, and a halation region in the Bs image signal, the Gs image signal, and the Rs image signal is detected by the halation detection unit. In a case where the halation region is detected, it is preferable to issue a warning display on the monitor 18. As the warning display, for example, there is a guidance display for instructing a user to keep the observation distance away. Additionally, in a case where the halation region is detected, it is preferable that the index value calculation unit 84 calculates the index values on the basis of pixel values of regions other than the halation region.

In addition, in the above embodiment, the blood vessel density, the blood vessel area, the blood vessel complexity, the blood vessel thickness, and the blood vessel length have been shown as specific examples of the index values. However, other index values may be used. As the index values of the blood vessels, for example, the number of pieces, the number of branches, a branch angle, a distance between branch points, the number of intersections, a change in thickness, intervals, depth with a mucous membrane as a reference, a height difference, inclination, contrast, color, a change in color, the degree of meandering, blood concentration, an artery proportion, a vein proportion, the concentration of an input coloring agent, a traveling pattern, blood flow rate, and the like, in terms of the blood vessels.

The number of blood vessels is the number of blood vessels extracted within the overall endoscopic image or the region of interest. The number of blood vessels is calculated, for example, using the number of branch points (the number of branches) of the extracted blood vessels, the number of intersection points (the number of intersections) with other blood vessels, or the like. The branch angle of blood vessels is an angle that two blood vessels form at a branch point. The distance between the branch points is a linear distance between an arbitrary branch point and its adjacent branch point, and a length along a blood vessel from the arbitrary branch point and its adjacent branch point.

The number of intersections of blood vessels is the number of intersection points where blood vessels with different depths under the mucous membrane intersect each other on the endoscopic image. More specifically, the number of intersections of blood vessels is the number of times with which blood vessels at a relatively shallow position under the mucous membrane intersect blood vessels at a relatively deep position.

The change in the thickness of the blood vessels is blood vessel information on variations in the thickness of the blood vessel, and is also referred to as the degree of aperture inequality. The change in thickness of blood vessels is, for example, the change rate (also referred to as the degree of dilation) of the vessel diameter. The change rate of the blood vessel diameter is calculated from "Change rate (%) of blood vessel diameter = Minimum diameter/Maximum diameter x 100", using the thickness (minimum diameter) of the thinnest portion of a blood vessel, and the thickness (maximum diameter) of a thickest portion of a blood vessel.

In addition, in a case where an endoscopic image obtained by imaging the observation target in the past inspection and an endoscopic image obtained by imaging the same observation target in subsequent new inspection, a time change in the thickness of the same blood vessel extracted from the endoscopic image obtained in the subsequent new inspection with respect to the thickness of the blood vessel extracted from the endoscopic image obtained in the past inspection may be used as the change in the thickness of the blood vessels.

Additionally, the percentage of a smaller-diameter part or the percentage of a larger-diameter part may be calculated as the change in the thickness of the blood vessels. The smaller-diameter part is a portion of which the thickness is equal to or smaller than a threshold value and the larger-diameter part is a portion of which the thickness is larger than the threshold value. The percentage of the smaller-diameter part is calculated from "Percentage (%) of smaller-diameter part = Length of smaller-diameter part/Length of blood vessel x 100". Similarly, the percentage of the larger-diameter part is calculated from "Percentage (%) of larger-diameter part = Length of larger-diameter part/Length of blood vessel x 100"

The interval of blood vessels is the number of pixels representing the mucous membrane between edges of extracted blood vessels. In a case where there is one extracted blood vessel, the interval of blood vessels does not have a value.

The depth of blood vessels is measured on the basis of the mucous membrane (more specifically, the surface of the mucous membrane). The depth of blood vessels with this mucous membrane as a reference can be calculated, for example, on the basis of the color of blood vessels. In the case of the special observation image, a blood vessel at a position near the surface of the mucous membrane is expressed in the magenta-based color and a blood vessel at a position distant from the surface of the mucous membrane and deep under the mucous membrane is expressed in the cyan-based color. Thus, the blood vessel information calculation unit 83 calculates the depth of the blood vessels with the mucous membrane as a reference for each pixel, on the basis of the balance between signals in respective colors of R, G, and B of pixels extracted as the blood vessels.

The height difference of blood vessels is the magnitude of a difference in depth of a blood vessel. For example, the height difference of one blood vessel to be observed is calculated from a height difference between the depth (the maximum depth) of the deepest location of this blood vessel, and the depth (minimum depth) of the shallowest location. The height difference is zero in a case where the depth is constant.

The inclination of blood vessels is the change rate of the depth of a blood vessel, and is calculated using the length of the blood vessel and the depth of the blood vessel. That is, the inclination of blood vessels is calculated from "Inclination of blood vessel = Depth of blood vessel/Length of blood vessel". In addition, a blood vessel may be divided into a plurality of sections, and the inclination of the blood vessel may be calculated in each section.

The contrast of the blood vessels is the contrast of the observation target with respect the mucous membrane. The contrast of the blood vessels is calculated from "Y_{V}/Y_{M}" or "(Y_{V} - Y_{M})/(Y_{V} + Y_{M})", using the luminance Y_{V} of blood vessels, and the luminance Y_{M} of the mucous membrane.

The color of blood vessels is respective values of RGB of the pixels representing the blood vessels. The change in the color of the blood vessels is a difference or a ratio between respective maximum and minimum values of respective RGB values of pixels representing blood vessels. For example, the ratio of a maximum value and a minimum value of pixel values of the B pixels representing the blood vessels, the ratio of a maximum value and a minimum value of pixel values of the G pixels, or the ratio of a maximum value and a minimum value of pixel values of the R pixels represents the change in the color of the blood vessels. Of course, the color of the blood vessels and the change in the color of the blood vessels may be calculated regarding respective values of cyan, magenta, yellow, green, and the like through conversion into complementary colors.

The degree of meandering of the blood vessels is blood vessel information representing the breadth of a range where blood vessels meander and travel. The degree of meandering of blood vessels is, for example, the area (number of pixels) of a minimum oblong shape including blood vessels from which the degree of meandering is calculated. Additionally, the ratio of the length of a blood vessel to a linear distance between a start point and an end point of the blood vessel may be used as the degree of meandering of blood vessels.

The blood concentration of the blood vessels is blood vessel information proportional to the amount of hemoglobin contained in the blood flowing through a blood vessel. Since the ratio (G/R) of the pixel values of the G values in the pixels to the pixel values of the R values in the pixels representing the blood vessels is proportional to the amount of hemoglobin, the blood concentration can be calculated for each pixel by calculating the value of G/R.

The degree of oxygen saturation of blood vessels is the amount of oxyhemoglobin to the total amount of hemoglobin (the total amount of oxyhemoglobin and reduced hemoglobin). The degree of oxygen saturation can be calculated using an endoscopic image obtained by imaging the observation target with light (for example, blue light with a wavelength of about 470 ± 10 nm) of a specific large wavelength range where a difference between the light absorption coefficients of the oxyhemoglobin and the reduced hemoglobin is large. Since the pixel values of the B pixels representing the blood vessels have a correlation with the degree of oxygen saturation in a case where the blue light with a wavelength of about 470 ± 10 nm is used, the degree of oxygen saturation of the respective pixels representing the blood vessels can be calculated by using a table or the like in which the pixel values of the B pixels are associated with the degree of oxygen saturation.

The artery proportion is the proportion of the number of pixels of arteries to the number of pixels of all blood vessels. Similarly, the vein proportion is the ratio of the number of pixels of veins to the number of pixels of all blood vessels. The arteries and the veins can be distinguished from each other depending on the degree of oxygen saturation. For example, if blood vessels with the degree of oxygen saturation of 70% or more are the arteries, and blood vessels with the degree of oxygen saturation of less than 70% are the veins, extracted blood vessels are divided into the arteries and the veins. Thus, the percentage of the above arteries and the percentage of the veins can be calculated.

The concentration of the input coloring agent is the concentration of the coloring agent dispersed onto the observation target, or the coloring agent injected into a blood vessel by intravenous injection. The concentration of the input coloring agent is calculated, for example, in the ratio of the pixel values of the color of the coloring agent to the pixel values of pixels other than the color of the coloring agent. For example, in a case where a coloring agent that gives blue color is input, the ratio B/G of the Bs image signal and the Gs image signal, the ratio B/R of the Bs image signal and the Rs image signal, or the like represents the concentration of the coloring agent fixed (or temporarily adhered) to the observation target.

The traveling pattern of the blood vessels is blood vessel information on the traveling direction of blood vessels. The traveling pattern of blood vessels is, for example, the average angle (traveling direction) of blood vessels to an arbitrarily set reference line, the dispersion (variations in traveling direction) of angles that blood vessels make with respect to the arbitrarily set reference line, or the like.

The blood flow rate (also referred to blood flow velocity) of the blood vessels is the number of red cells through which pass through a blood vessel per unit time. For example, in a case where an ultrasonic probe is used together via a forceps channel or the like of the endoscope 12, the blood flow rate of blood vessels can be calculated by calculating the Doppler shift frequency of the respective pixels representing the blood vessels of the endoscopic image, using signals obtained by the ultrasonic probe.

### [Second Embodiment]

In a second embodiment, the observation target is illuminated using a laser light source and a fluorescent body instead of the four-color LEDs 20a to 20d illustrated in the above first embodiment. In the following, only portions different from the first embodiment will be described, and description of substantially the same portions as those of the first embodiment will be omitted.

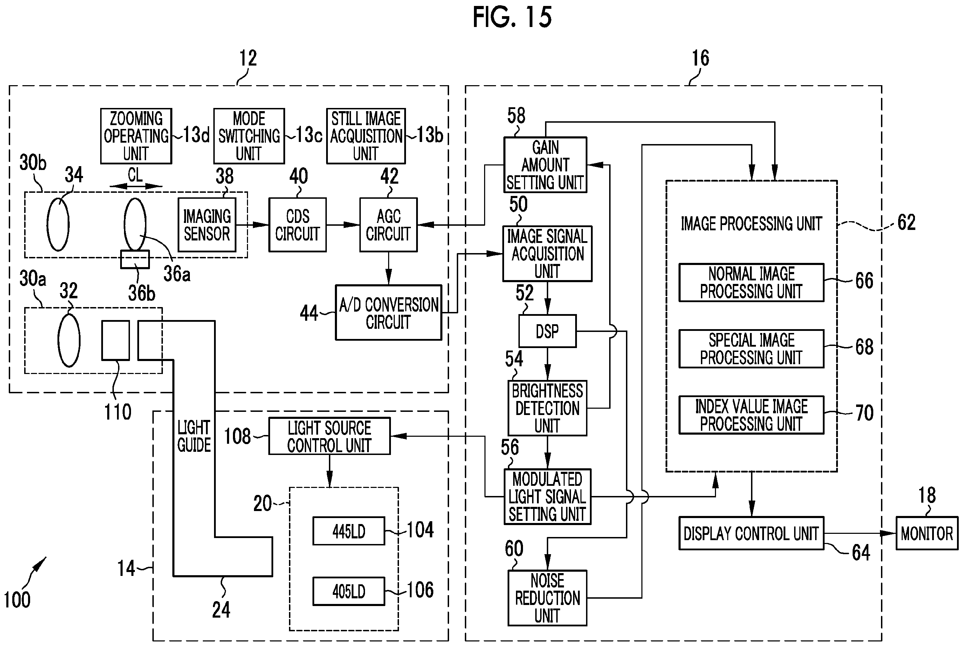

As illustrated in Fig. 15, in the endoscope system 100 of the second embodiment, in the light source 20 of the light source device 14, a blue laser light source that emits blue laser light having a central wavelength of 445 ± 10 nm (written as "445LD"; LD represents Laser Diode) 104 and a blue-violet laser light source (written as "405LD") 106 that emits blue-violet laser light having a central wavelength of 405 ± 10 nm are provided instead of the four-color LEDs 20a to 20d. The light emission from semiconductor light-emitting elements of the respective light sources 104 and 106 are individually controlled by a light source control unit 108, and the quantity-of-light ratio of the emitted light of the blue laser light source 104 and the emitted light of the blue-violet laser light source 106 is changeable.

The light source control unit 108 turns on the blue laser light source 104 in the case of the normal mode. In contrast, in the case of the special mode or the index value display mode, both the blue laser light source 104 and the blue-violet laser light source 106 are turned on, and the light emission ratio of the blue laser light is controlled to become larger than the light emission ratio of the blue-violet laser light.

In addition, it is preferable that the half width of the blue laser light or the blue-violet laser light is about ±10 nm. Additionally, as the blue laser light source 104 and the blue-violet laser light source 106, broad area type InGaN-based laser diodes can be utilized, and InGaNAs-based laser diodes or GaNAsb-based laser diodes can also be used. Additionally a configuration using a light emitter, such as a light-emitting diode, may be adopted as the above light source.

The illumination optical system 30a is provided with a fluorescent body 110 that the blue laser light or the blue-violet laser light from the light guide 24 enters in addition to the illumination lens 32. The fluorescent body 110 is excited by the blue laser light to emit fluorescence. Additionally, a portion of the blue laser light is transmitted through the fluorescent body 110 without exciting the fluorescent body 110. The blue-violet laser light is transmitted through the fluorescent body 110 without exciting the fluorescent body 110. The inside of the body of the observation target is illuminated with the light emitted from the fluorescent body 110 via the illumination lens 32.

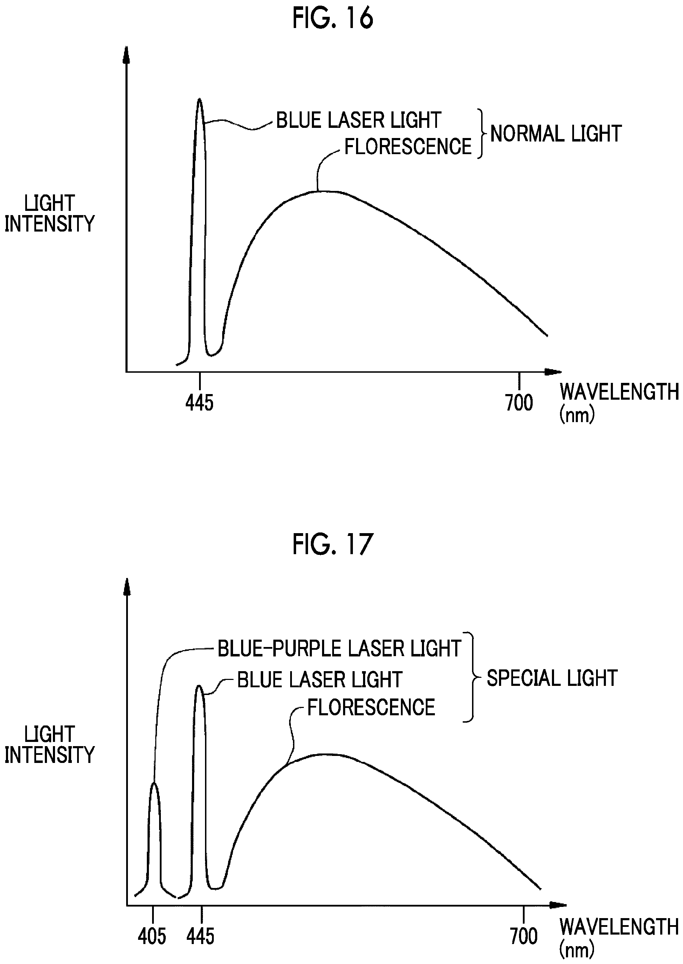

Here, in the normal mode, mainly, the blue laser light enters the fluorescent body 110. Therefore, the broadband light for normal mode, which is obtained by combining the blue laser light with the fluorescence excited and emitted from the fluorescent body 110 due to the blue laser light as illustrated in Fig. 16, is illuminated to the observation target as the normal light. By imaging the observation target illuminated with the normal light by the imaging sensor 38, the normal image including the Bc image signal, the Gc image signal, and the Rc image signal is obtained.

On the other hand, in the special mode, the blue-violet laser light and the blue laser light enter the fluorescent body 110. Therefore, the broadband light for special mode, which is obtained by combining the blue-violet laser light, the blue laser light, and the fluorescence excited and emitted from the fluorescent body 110 due to the blue laser light together as illustrated in Fig. 17, is illuminated to the observation target as the special light. By imaging the observation target illuminated with the special light by the imaging sensor 38, the special image including the Bs image signal, the Gs image signal, and the Rs image signal is obtained. Additionally, in the index value display mode, the observation target illuminated with the special light is imaged by the imaging sensor 38, and Bs image signal, Gs image signal, and Rs image signal are acquired, the index values are calculated on the basis of the three image signals, and the index value image is generated.

In addition, as the fluorescent body 110, it is preferable to use those configured to include a plurality of types of fluorescent bodies (for example, a YAG-based fluorescent body or fluorescent bodies, such as BAM (BaMgAl₁₀O₁₇)) that absorb a portion of the blue laser light and are excited to emit light in green to yellow. As in the present embodiment, in a case where a semiconductor light-emitting element is used as an excitation light source of the fluorescent body 110, the white light having higher intensity are obtained with high light emission efficiency. Additionally, in a case where a semiconductor light-emitting element is used as an excitation light source of the fluorescent body 110, the intensity of the white light can be easily adjusted. Moreover, in a case where a semiconductor light-emitting element is used as an excitation light source of the fluorescent body 110, changes in the color temperature and chromaticity of the white light can be suppressed to be small.

### [Third Embodiment]

In the third embodiment, the observation target is illuminated using a white light source, such as a xenon lamp, and the rotation filter instead of the four-color LEDs 20a to 20d. Additionally, the observation target may be imaged by a monochrome imaging sensor instead of the color imaging sensor 38. In the following, only portions different from the first embodiment will be described, and description of substantially the same portions as those of the first embodiment will be omitted.

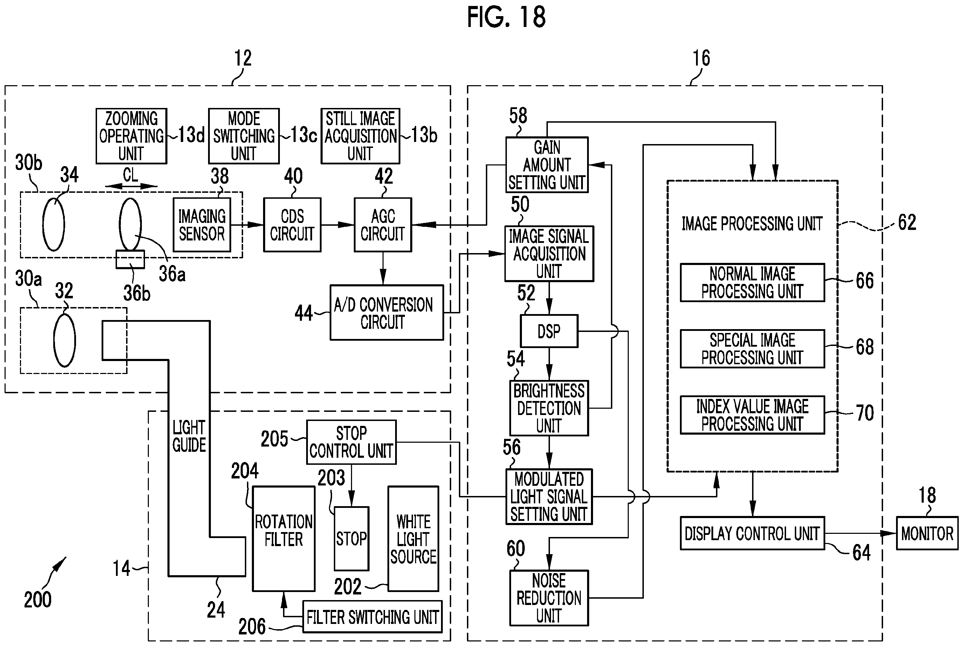

In an endoscope system 200 illustrated in Fig. 18, in the light source device 14, a white light source 202, a rotation filter 204, and a filter switching unit 206 are provided instead of the respective LEDs 20a to 20d of the endoscope system 10. Additionally, the imaging optical system 30b is provided with a monochrome imaging sensor 208, which is not provided with a color filter, instead of the color imaging sensor 38. Additionally, a stop 203 is provided between the white light source 202 and the rotation filter 204, and the area of an opening of the stop 203, is adjusted by a stop control unit 205. The stop control unit 205 controls the stop 203 on the basis of the modulated light signal from the processor device 16.

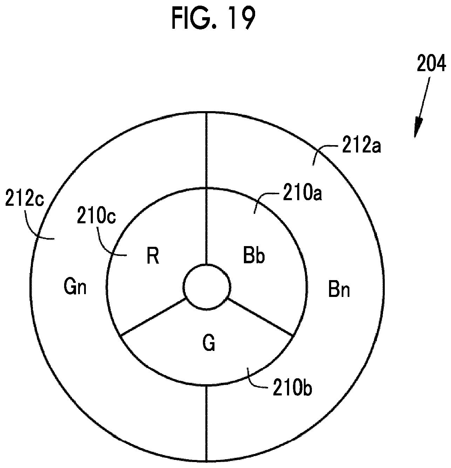

The white light source 202 is a xenon lamp, a white LED, or the like, and emits white light of which the wavelength range ranges from blue to red. The rotation filter 204 comprises a normal mode filter 210 that is provided on an inner side closest to a rotation axis thereof, and a special mode or index value display mode filter 212 provided outside the normal mode filter 210 (refer to Fig. 19).

The filter switching unit 206 moves the rotation filter 204 in a radial direction. Specifically, the filter switching unit 206 inserts the normal mode filter 210 into a white light path in a case where the normal mode is set by the mode switching unit 13c. Specifically, the filter switching unit 206 inserts the special mode or index value display mode filter 212 into the white light path in a case where the special mode or the index value display mode is set by the mode switching unit 13c.

As illustrated in Fig. 19, a Bb filter 210a, a G filter 210b, and an R filter 210c are provided in the circumferential direction in the normal mode filter 210. The Bb filter 210a transmits the broadband blue light Bb, which has a wavelength range of 400 to 500 nm, in the white light. The G filter 210b transmits the green light G in the white light. The R filter 210c transmits the red light R in the white light. Hence, in the normal mode, as the rotation filter 204 rotates, the broadband blue light Bb, the green light G, and the red light R are sequentially radiated toward the observation target as the normal light.

A Bn filter 212a and a Gn filter 212b are provided in the circumferential direction in the special mode or index value display mode filter 212. The Bn filter 212a transmits narrowband blue light Bn of 400 to 450 nm in the white light. The Gn filter 212b transmits narrowband green light Gn of 530 to 570 nm in the white light. Hence, in the special mode, as the rotation filter 204 rotates, the narrowband blue light Bn and the narrowband green light Gn are sequentially radiated toward the observation target as the special light.

In the endoscope system 200, in the normal mode, whenever the observation target is illuminated with the broadband blue light Bb, the green light G, and the red light R, respectively, the observation target is imaged by the monochrome imaging sensor 208. As a result, the Bc image signal is obtained at the time of the illumination with the broadband blue light Bb, the Gc image signal is obtained at the time of the illumination with the green light G, and the Rc image signal is obtained at the time of the illumination with the red light R. The normal image is constituted of the Bn image signal, the Gc image signal, and the Rc image signal.

In the special mode, the observation target is imaged by the monochrome imaging sensor 208 whenever the observation target is illuminated with narrowband blue light Bn and the narrowband green light Gn, respectively. Accordingly, the Bn image signal is obtained at the time of the illumination with the narrowband blue light Bn, and the Gn image signal is obtained at the time of the irradiation with the narrowband green light Gn. The special image is constituted of the Bn image signal and the Gn image signal. Additionally, in the case of the index value display modes, the calculation of the index values and the generation of the index value image are performed on the basis of the Bn image signal obtained at the time of the illumination with the narrowband blue light Bn, and the Gn image signal obtained at the time of the illumination with the narrowband green light Gn. In addition, in the index value display mode, it is preferable that the brightness detection unit 54 detects the brightness of the observation target on the basis of the Gn image signal.

In the above-described respective embodiments, the AGC circuit 42 is provided in the endoscope 12. However, the AGC circuit 42 may be provided in the processor device 16.

In the above-described respective embodiments, hardware structures of the processing units, which executes various kinds of processings, such as the image signal acquisition unit 50, the brightness detection unit 54, the modulated light signal setting unit 56, the gain amount setting unit 58, the noise reduction unit 60, the image processing unit 62, the display control unit 64, and of the AGC circuit 42, are various kinds of processors as shown below. Various processors include exclusive electric circuits, which are processors having circuit configurations exclusively designed to execute specific processing, such as a central processing unit (CPU) that is a general-purpose processor that executes software (programs) to function as various processing units, a programmable logic device (PLD) that is a processor capable of changing a circuit configuration after manufacture of a field programmable gate array (FPGA) or the like, and an application specific integrated circuit (ASIC).

One processing unit may be constituted of one of these various processors, or may be constituted of two or more same or different processors (for example, a plurality of the FPGAs or a combination of the CPU and the FPGA). Additionally, the plurality of processing units may be composed of one processor. As an example in which the plurality of processing units are constituted of the one processor, firstly, as represented by a computer, such as a client or a server, there is a form in which one processor is constituted of a combination of one or more CPUs and software and this processor functions as a plurality of processing units. Secondly, as represented by a system-on-chip (SOC) or the like, there is a form in which a processor, which realizes functions of an overall system including the plurality of processing units with one integrated circuit (IC) chip, is used. In this way, the various processing units are configured by using one or more of the above various processors as the hardware structure(s).

Moreover, the hardware structures of these various processors are more specifically circuitries in which circuit elements, such as semiconductor elements, are combined together.

### Explanation of References

10: endoscope system
12: endoscope
12a: insertion part
12b: operating part
12c: bending part
12d: distal end part
13a: angle knob
13b: still image acquisition unit
13c: mode switching unit
13d: zooming operating unit
14: light source device
16: processor device
18: monitor
19: console
20: light source
20a: V-LED
20b: B-LED
20c: G-LED
20d: R-LED
22: light source control unit
23: wavelength cutoff filter
24: light guide
30a: illumination optical system
30b: imaging optical system
32: illumination lens
34: objective lens
36: magnifying optical system
36a: zoom lens
36b: lens drive unit
38: imaging sensor
40: CDS circuit
42: AGC circuit
44: A/D conversion circuit
50: image signal acquisition unit
52: DSP
54: brightness detection unit
56: modulated light signal setting unit
58: gain amount setting unit
60: noise reduction unit
62: image processing unit
64: display control unit
66: normal image processing unit
68: special image processing unit
70: index value image processing unit
80: clarity calculation unit
82: index value selection unit
83: blood vessel information calculation unit
84: index value calculation unit
86: index value image generation unit
90: noise amount calculation unit
90a: noise amount conversion table
94: observation distance calculation unit
100: endoscope system
104: blue laser light source
106: blue-violet laser light source
108: light source control unit
110: fluorescent body
200: endoscope system
202: white light source
203: stop
204: rotation filter
205: stop control unit
206: filter switching unit
208: imaging sensor
210: normal mode filter
210a: Bb filter
210b: G filter
210c: R filter
212: special mode or index value display mode filter
212a: Bn filter
212b: Gn filter

## Claims

1. A processor device comprising:
an image acquisition unit that acquires an image obtained by imaging an observation target including a structure;
a clarity calculation unit that calculates a clarity of the structure on the basis of the image;
an index value selection unit that selects an index value capable of being calculated on the basis of the clarity of the structure calculated by the clarity calculation unit among a plurality of index values; and
an index value calculation unit that calculates the index value selected by the index value selection unit, on the basis of the image.

2. The processor device according to claim 1,
wherein the clarity of the structure is expressed by a noise amount of the image,
wherein the clarity calculation unit has a noise amount calculation unit that calculates the noise amount, and
wherein the index value selection unit selects an index value capable of being calculated from the image having the noise amount among the plurality of index values.

3. The processor device according to claim 2, further comprising:
a gain processing unit that performs a gain processing on the image,
wherein the noise amount calculation unit calculates the noise amount from a gain amount to be used in the gain processing.

4. The processor device according to claim 2,
wherein the noise amount calculated by the noise amount calculation unit is changed in accordance with a modulated light signal to be used for control of a light source in a case where a quantity of light emission of the light source that emits the light with which the observation target is illuminated exceeds a specific quantity of light emission.

5. The processor device according to any one of claims 2 to 4,
wherein the noise amount calculation unit calculates the noise amount from a spatial change amount of a pixel value of the image.

6. The processor device according to any one of claims 2 to 4,
wherein the noise amount calculation unit calculates the noise amount from a temporal change amount of a pixel value of the image.

7. The processor device according to any one of claims 2 to 6,
wherein the index value selection unit selects an index value for a high noise including an index value relating to a low-frequency structure in a case where the noise amount exceeds a specific noise amount threshold value, and selects an index value for a low noise including an index value relating to a high-frequency structure in a case where the noise amount is equal to or less than the specific noise amount threshold value.

8. The processor device according to claim 7,
wherein the index value for a high noise includes an area of the structure or a density of the structure, and
wherein the index value for a low noise includes a thickness of the structure or a length of the structure.

9. The processor device according to claim 1,
wherein the clarity of the structure is expressed by an observation distance indicating a distance from the observation target,
wherein the clarity calculation unit has an observation distance calculation unit that calculates the observation distance on the basis of the image, and
wherein the index value selection unit selects an index value capable of being calculated from the image obtained in the calculated observation distance among the plurality of index values.

10. The processor device according to claim 9,
wherein the index value selection unit selects an index value for a first observation distance including an index value relating to a low-frequency structure in a case where the observation distance is a first observation distance exceeding a specific observation distance threshold value, and selects an index value for a second observation distance including an index value relating to a high-frequency structure in a case where the observation distance is a second observation distance equal to or less than the specific observation distance threshold value.

11. The processor device according to claim 10,
wherein the index value for the first observation distance includes an area of the structure or a density of the structure, and
wherein the index value for the second observation distance includes a thickness of the structure or a length of the structure.

12. The processor device according to any one of claims 1 to 11,
wherein the structure is a blood vessel structure or a glandular structure.

13. A processor device comprising:
an image acquisition unit that acquires an image obtained by imaging an observation target including a structure;
a noise amount calculation unit that calculates a noise amount of the image on the basis of the image;
an index value selection unit that selects an index value capable of being calculated from the image having the noise amount among a plurality of index values, the index value selection unit selecting an index value for a high noise including an index value relating to a low-frequency structure in a case where the noise amount exceeds a specific noise amount threshold value, and selecting an index value for a low noise including an index value relating to the high-frequency structure in a case where the noise amount is equal to or less than the specific noise amount threshold value; and
an index value calculation unit that calculates an index value selected by the index value selection unit, on the basis of the image.

14. An endoscope system comprising:
an image acquisition unit that acquires an image obtained by imaging an observation target including a structure;
a clarity calculation unit that calculates a clarity of the structure on the basis of the image;
an index value selection unit that selects an index value capable of being calculated on the basis of the clarity of the structure calculated by the clarity calculation unit among a plurality of index values;
an index value calculation unit that calculates the index value selected by the index value selection unit, on the basis of the image;
an index value image generation unit that generates an index value image obtained by imaging the index value; and
a display unit that displays the index value image.

15. A method of operating a processor device comprising the steps of:
acquiring an image obtained by imaging an observation target including a structure, using an image acquisition unit;
calculating a clarity of the structure on the basis of the image, using a clarity calculation unit;
selecting an index value capable of being calculated on the basis of the clarity of the structure calculated by the clarity calculation unit among a plurality of index values, using an index value selection unit; and
calculating the index value selected by the index value selection unit, on the basis of the image, using an index value calculation unit.
